# EUROPEAN PATENT SPECIFICATION

(11) **EP 0 504 290 B1**
(45) Date of publication and mention of the grant of the patent: **22.01.1997**
(21) Application number: 91901399.5
(22) Date of filing: 07.12.1990
(51) Int. Cl.: A01N 43/66

(54) **INHIBITORS OF PNEUMOCYSTIS CARINII DIHYDROFOLATE REDUCTASE**
INHIBITOREN DER DIHYDROFOLATREDUKTASE VON PNEUMOCYSTIS CARINII
INHIBITEURS DE REDUCTASE DE DIHYDROFOLATE DE PNEUMOCYSTIS CARINII

(30) Priority: 07.12.1989 US 447181
(43) Date of publication of application: 23.09.1992
(73) Proprietor: CHIRON CORPORATION, Emeryville California 94608-2916 (US)
(72) Inventor: SANTI, Daniel, V., San Francisco, CA 94117 (US); MARLOWE, Charles, K., San Francisco, CA 94118 (US)
(74) Representative: Glawe, Delfs, Moll & Partner
(86) International application number: US9007218
(87) International publication number: WO9108668

(56) References cited:
- US-A- 2 823 161
- US-A- 2 836 539
- US-A- 2 959 519
- US-A- 3 074 947
- US-A- 3 123 527
- US-A- 3 215 600
- US-A- 3 959 469
- ANTIMICROBIAL AGENTS AND CHEMOTHERAPY, vol. 33, no. 11, November 1989, pages 1860-1863, American Society for Microbiology; J.D. BERMAN et al.: "Antileishmanial activities of 2,4-diaminoquinazoline putative dihydrofolate reductase inhibitors"
- IDEM
- ANTIMICROBIAL AGENTS AND CHEMOTHERAPY, vol. 32, no. 4, April 1988, pages 430- 433, American Society for Microbiology; J.A. KOVACS et al.: "Potent antipneumocystis and antitoxoplasma activities of piritrexim, a lipid-soluble antifolate"
- E. MUTSCHLER: "Arzneimittelwirkungen, Lehrbuch der Pharmakologie und Toxikologie", 5th edition, 1986, page 665, Wissenschaftliche Verlagsgesellschaft mbH, Stuttgart, DE
- E. MUTSCHLER: "Arzneimittelwirkungen, Lehrbuch der Pharmakologie und
- Toxikologie", 5th edition, 1986, pages 586-592, Wissenschaftliche Verlagsgesellschaft mbH, Stuttgart, DE
- "The Merck Index", 10th edition, 1983, page 390, no. 2715, Merck & Co., Inc., Rahway, NJ, US
- ARCHIVES OF BIOCHEMISTRY AND BIOPHYSICS, vol. 266, no. 1, October 1988, pages 152-161, Academic Press, Inc.; M.G. KOEHLER et al.: "A molecular shape analysis and quantitative structure-activity relationship investigation of some triazine-antifolate inhibitors of Leishmania dihydrofolate reductase"
- THE JOURNAL OF INFECTION, vol. 22, no. 2, March 1991, pages 143-152, The British Society for the Study of Infection; A. GONZALEZ-RUIZ et al.: "Dapsone in low doses prevents Pneumocystis carinii pneumonia in the rat model"
- Avery's Drug Treatment, Principles and Practices of Clinical Pharmacology and Therapeutics 3rd Edition, TREVOR M SPEIGHT ed, William and Wilkins, Baltimore.
- The Menck Manual of Diagnosis and Therapy 14th edition, ROBERT BERKOW ed, Menck Sharp and Dohme Research Laboratories, Rahway N.J. 1982.

## Description

### Technical Field

This invention is directed to methods of treating P. carinii pneumonia using inhibitors of dihydrofolate reductase (DHFR).

### Background

Pneumocystis carinii pneumonia is a leading cause of morbidity and mortality in acquired immuno-deficiency syndrome (AIDS). Since the onset of the AIDS epidemic, the incidence of P. carinii pneumonia has risen from approximately 200 cases per year to greater than 25,000 cases per year in the United States.

Due to the lack of a continuous in vitro culture system and the cumbersome nature of the rat model of P. carinii pneumonia, anti-P. carinii therapy has been developed largely on the assumption that antiprotozoan agents were likely to be effective. In fact, P. carinii has recently been shown to be a member of the Fungi. The two principal therapeutic modalities, trimethoprim/sulfamethoxazole and pentamidine, were developed using the anti-protozoan theory.

Trimethoprim and pyrimethamine, and other dihydrofolate reductase (DHFR) inhibitors (such as methotrexate), are known to be effective antineoplastic, anti-bacterial, and anti-protozoal agents because of the central role played by DHFR in the de novo synthesis of nucleic acid precursors. Despite their obvious efficacy when used in conjunction with a sulfonamide, trimethoprim and pyrimethamine are in themselves poor inhibitors of P. carinii DHFR [50% inhibitory concentration values (IC50) of 39,600 and 2,400 nM respectively compared to 8 and 2,50O nM for E. coli DHFR at similar substrate concentrations]. Other antifolates have been shown to be more effective inhibitors of P. carinii DHFR, but require concomitant administration of leucovorin to prevent host toxicity.

Prior to the AIDS epidemic, these types of agents were sufficient for treatment of the rare cases of P. carinii pneumonia. However, in the HIV-positive patient, therapy and prophylaxis with the standard anti-P. carinii agents are complicated by frequent toxic and allergic side effects. New compounds that surpass the efficacy of the known antifolates in treating pneumocystis pneumonia are desirable, especially inhibitors with greater selectivity for P. carinii DHFR relative to host (especially human) DHFR than that which exists for known inhibitors such as trimethoprim.

### Relevant Literature

A number of l-aryl-s-triazine compounds and their use in treating malaria are discussed in U.S. Patent No. 3,074,947. The anti-malarial compound Nl-p-chlorophenyl-N5-isopropylbiguanide, also known as proguanil, which is converted to cycloguanil in vivo, is described along with a method for its production in U.S. Patent No. 2,529,992. Quantitative structure-activity relationships of triazine-antifolate inhibition of Leishmania dihydrofolate reductase and cell growth, along with synthetic techniques for some of the compounds used, are discussed in Boot™ et al., J. Med. Chem. (1987) 30:1218-1224.

### SUMMARY OF THE INVENTION

It is an object of the present invention to provide a method of treating or preventing P. carinii pneumonia using a dihydrofolate reductase inhibitor that is more selective for P. carinii DHFR relative to host DHFR, especially human DHFR, or has a higher affinity for P. carinii DHFR, than currently known DHFR inhibitors such as trimethoprim.

This and other objects of the invention have been accomplished by providing a method of selectively inhibiting P. carinii dihydrofolate reductase in the presence of mammalian dihydrofolate reductase by administering a l-phenyl-s-triazine to a host infected with P. carinii. l-Phenyl-s-triazines are known compounds that are effective against malaria but that have not previously been used in the treatment of P. carinii pneumonia. Particularly preferred are triazines of the formula: 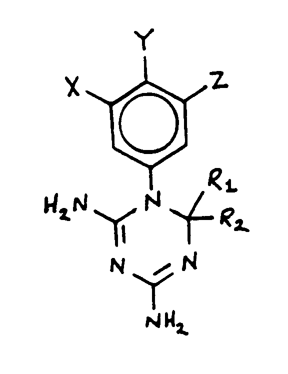 wherein:
X, Y, and Z are independently selected from the group consisting of hydrogen, hydroxy, halogen, aryl lower alkyl, lower alkyl, lower alkylthio, lower alkyloxy, and trifluoromethyl or one of X and Y is selected from the group consisting of CO₂R (where R is lower alkyl), -OCH₂Cl, -OCH₃, and a radical containing at least 3 carbon atoms and being of the formula -A₁-(CH₂)n-A₂-R₃, wherein A₁ is a bond, O, or S; n is an integer from 1 to 12 inclusive; A₂ is a bond, O, S, Se, or NH; and R₃ is selected from the group consisting of methyl, adamantyl, naphthyl, phenyl, and phenyl substituted with 1 to 3 radicals selected from the group consisting of halo, lower alkyl of 1 to 6 carbon atoms, trifluoromethyl, lower alkoxy, cyano, acetamido, ureido, phenyl, and amido;
Rₗ is lower alkyl; and
R₂ is hydrogen or methyl;
and compounds which are amides or acid addition salts formed by reaction an organic or inorganic acid with a triazine of said formula. Treatment with a precursor of a l-phenyl-s-triazine, such as proguanil, which is known to be converted in vivo to cycloguanil, a known antimalarial l-phenyl-s-triazine, is equivalent to treatment with a l-phenyl-s-triazine itself.

### DESCRIPTION OF THE DRAWINGS

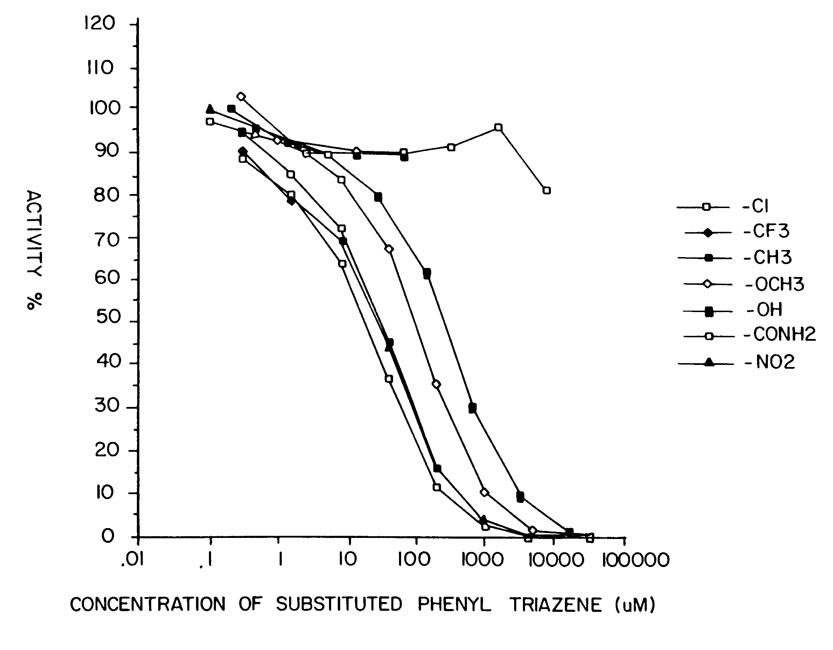

The Figure is a graph showing effects of a series of differently substituted triazines on P. carinii DHFR activity.

### DESCRIPTION OF SPECIFIC EMBODIMENTS

The present invention provides a method for inhibiting growth of Pneumocystis carinii in a mammalian host by administering to the host an amount of a l-phenyl-s-triazine effective to inhibit growth of Pneumocystis carinii. l-Phenyl-s-triazines are compounds known to be effective as antimalarial agents. However, they had not been used in the treatment P. carinii pneumonia prior to the present invention.

Experiments in the laboratory of the inventor have demonstrated that a number of different l-phenyl-s-triazine compounds are selective in their ability to inhibit dihydrofolate reductase of P. carinii relative to mammalian DHFR. For example, a number of compounds of the invention are selective for P. carinii DHFR (PcDHFR) over human DHFR as indicated by a low Kᵢ for PcDHFR relative to human. See the Table that appears below in the Examples for data on specific compounds. However, even compounds of the invention that bind more tightly to human DHFR relative to PcDHFR show improvement over agents currently used to treat P. carinii infections. For example, the anti-malarial compound cycloguanil, which is 4,6-diamino-1-(p-chlorophenyl)1,2-dihydro-2,2-dimethyl-s-triazine, shows a Kᵢ of 109 nM for P. carinii DHFR, whereas its Kᵢ for human DHFR is 4300 nM. Thus, cycloguanil binds some 2.5 times more tightly to human DHFR than to P. carinii DHFR.

In contrast, trimethoprim, the antifolate most widely used to treat P. carinii infections, is a poor inhibitor of the P. carinii enzyme and shows a greater specificity for human DHFR. Trimethoprim has a Kᵢ of 280 µM for P. carinii DHFR and a Kᵢ of 48 µM for human DHFR. It therefore shows reverse selectivity for DHFR of the infective organism about twice as poor as cycloguanil, while obviously being less selective than the Pc-selective compounds of preferred embodiments.

Accordingly, cycloguanil and related l-phenyl-s-triazines and their precursors, either alone or in combination with other agents, will be more effective than trimethoprim in treating mammalian infections by Pneumocystis carinii because of their ability to selectively inhibit P. carinii DHFR in the presence of mammalian, especially human, DHFR.

Compounds used in the method of the invention are l-phenyl-s-triazines and are particularly preferred to be 4,6-diamino-1,2-dihydro-1-phenyl-s-triazines as well as amides and salts formed by the reaction of an inorganic or organic acid with an amino group of the triazine. Such compounds typically have the formula: 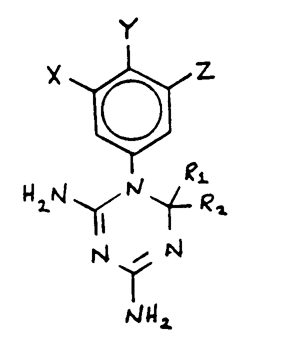 wherein:
X, Y, and Z are independently selected from the group consisting of hydrogen, hydroxy, halogen, alkyl, aryl alkyl, alkylthio, alkyloxy, and trifluoromethyl (as well as any of these alkyl groups functionalized with one or more organic substituents named in this application);
Rl is lower alkyl; and
R2 is hydrogen or methyl.

As used in the foregoing definitions, halogen designates a fluorine, chlorine, bromine, or iodine atom. Alkyl means linear or branched alkyl containing from 1 to 15 carbons in preferred embodiments. Lower alkyl here and elsewhere preferably means a linear or branched alkyl group containing from 1 to 5 carbon atoms. Aryl groups are preferably carbocyclic or heterocyclic systems containing one or two aromatic rings (when two ring, preferably fused). Examples of suitable aryl rings include benzene, furan, thiophene, pyrrole, pyrrazole, triazole, isoxazole, oxazole, thiazole, isothiazole, pyran, pyrone, dioxin, pyridine, pyridizine, purimidine, pyrazine, triazine, indene, benzofuran, isobenzofuran, benzothiofuran, indole, napthalene, coumarin, quinoline, and isoquinoline. Simple aryl groups such as phenyl, napthyl, and single-ring heterocycles are preferred. Phenyl is particularly preferred. Preferred aryl lower alkyl groups are those in which the aryl and lower alkyl substituents have the previously defined meanings. Benzyl is a particularly preferred aryl lower alkyl group. In all cases, normal substituents found on aromatic rings, such as halogen, amino, hydroxy, and amido groups, can be present and are included within the meaning of aryl. However, aryl groups substituted only with hydrogens are preferred. Attachment of X, Y, or Z to the phenyl group of the 1-phenyl-s-triazine preferably occurs through a -CH₂-, -O-, or -S- linkage. Branching at the point of attachment (as in, e.g., t-butyl) or hybridization other than sp³ for the atom attached to the phenyl ring is less preferred.

Particularly preferred are compounds in which Y is hydrogen, halogen, methyl, or trifluoromethyl. Also preferred are compounds in which at least one of X, Y, and Z is hydrogen; especially preferred are compounds in which both X and Z are hydrogen, particularly when Y is chloro, trifluoromethyl, methyl, methoxy, or hydroxy. Preferred groups for Rl are methyl and ethyl, and R₁ and R₂ both being methyl is particularly preferred. Specific preferred compounds include cycloguanil and the related compounds in which the chlorine on the phenyl ring of cycloguanil is replaced by a trifluoromethyl, methyl, methoxy, or hydroxy group.

Another group preferred compounds includes those in which R₁ and R₂ are each methyl and either X and Y are both chloro or one of X and Y is selected from the group consisting of CO₂R (where R is lower alkyl), -OCH₂Cl, -OCH₃, and a radical containing at least 3 carbon atoms and being of the formula -A₁-(CH₂)ₙ-A₂-R₃, wherein A₁ is a bond, O, or S; n is an integer from 1 to 12 inclusive; A₂ is a bond, O, S, Se, or NH; and R₃ is selected from the group consisting of methyl, adamantly, naphthyl, phenyl, and phenyl substituted with 1 to 3 radicals selected from the group consisting of halo, lower alkyl of 1 to 6 carbon atoms, trifluoromethyl, lower alkoxy, cyano, acetamido, ureido, phenyl, and amido.

A group of specific preferred compounds consists of all compounds in Table 2 of the following examples that have a selectivity (defined as human Kᵢ/PcKᵢ) of at least 0.5; a more preferred specific group consists of those compounds having a selectivity of at least 0.9; an even more preferred group has a selectivity of at least 2.0.

The 4,6-diamino-1,2-dihydro-2-lower alkyl-l-aryl-s-triazines and their soluble salts used in the practice of the invention can be prepared in various ways. They can be obtained by reaction a l-arylbiguanide of the formula 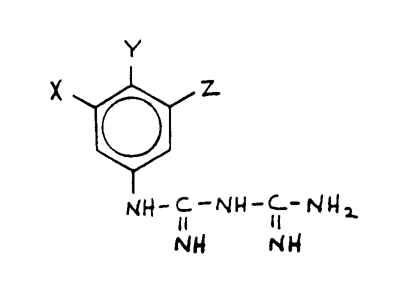 with a lower aliphatic aldehyde or ketone of the formula 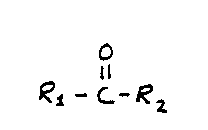 in the presence of a strong acid, where X, Y, Z, Rl and R2 are as defined before. The l-arylbiguanides can be obtained by the reaction of an aniline derivative of the formula 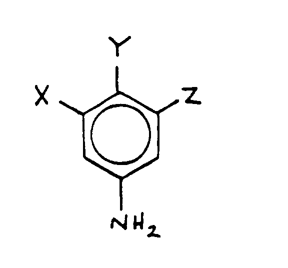 with dicyandiamide in the presence of a strong acid; where X, Y and Z are as defined before. Alternatively, the aniline derivative, the aldehyde or ketone, and dicyandiamide are reacted in the presence of a strong acid and the desired triazine derivative is obtained directly. This method is preferred in those case where Rₗ and R₂ are both lower alkyl groups. The desired triazine derivative is obtained directly from the reaction mixture as an acid-addition salt or as the free base following basification.

In addition to the free triazines described above, salts and amides formed by reaction of an inorganic or organic acid with one of the free amino groups can be used in the practice of the invention. Examples of inorganic acids include hydrochloric, hydrobromic, and sulfuric acids. Examples of organic acids include any of a number of carboxylic acids, such as acetic acid and various fatty acids. The pamoic acid salts are particularly preferred as such salts have been shown to be effective in providing long-acting forms of triazines in the treatment of other diseases. See, for example, U.S. Patent No. 3,074,947.

Administration of a compound that acts as precursor of one of the triazines described above is considered to be equivalent to administration of the triazine itself. For example, the compound proguanil, which is l-p-chlorophenyl-5-isopropylbiguanide, had been shown to be slowly converted to cycloguanil (see above) on administration to mammalian hosts. Most precursors are biguanides of the formula: 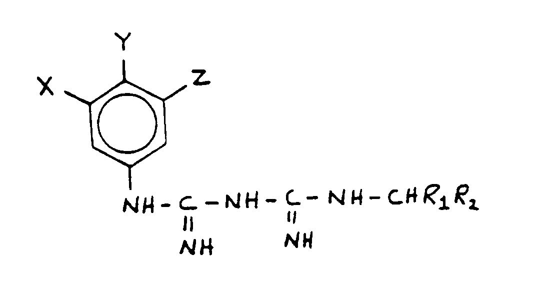 in which X, Y, Z, Rl, and R2 have the meaning defined above. Biguanides can be prepared by the general reactions described above. See, for example, Kurd et al., J. Chem. Soc. 1946: 729 and Kurd et al., Ibid. 948: 1630.

The method of the invention contemplates using compounds as described above either alone or in combination with a second (or further) compound to provide enhanced effectiveness against P. carinii. The second compound used is typically one which is known (l) to potentiate activity of a dihydrofolate reductase inhibitor in Pneumocystis carinii or (2) to counter or circumvent the action of a dihydrofolate reductase inhibitor in the mammalian host. For example, a sulfonamide or dapsone can be used to potentiate the effect of the DHFR inhibitor. Such combinations are known in treatment of other parasitic organisms, such as in malaria. For example, the combination of trimethoprim and a sulfonamide such as sulphamethoxazole has been used in treating gram negative bacteria such as E. coli under the tradenames Septra and Bactrim, and combination of a triazine and a sulfonamide should likewise be useful in the present case. Alternatively, citrovorum factor (CF; also known as Leucovorin; 5-formyl-5,6,7,8-tetrahydrofolic acid) is known to reverse the effect of DHFR inhibitors by providing a direct source of reduced folates. CF has been commonly used to reverse the effects of methotrexate in treatment of certain types of cancer. However, CF does not enter Pneumocystis carinii cells, so it can rescue host cells from the effect of a P. carinii DHFR inhibitor without rescuing the P. carinii cells themselves. This principle has been previously applied in the treatment of P. carinii pneumonia with non-specific antifolates such as Trimetrexate and Pyritrexim, which potently inhibit human DHFR. Coadministration of citrovorum factor with a triazine such as cycloguanil will permit higher doses or longer durations to be used without toxicity caused by inhibition of human DHFR than would be possible in the absence of the reduced folate source. Other sources of tetrahydrofolates can be used in the place of CF. 5-Methyl THF is an example of a different source of reduced folate.

The compositions according to the invention may be administered per os or parenterally in an amount sufficient to inhibit growth of the organism. Death of the organisms is not required, but is preferred. The dose is adjusted as required to avoid unnecessary toxicity to the host. Initial treatment usually will begin with doses ranging from 0.1 to 30 mg/kg body weight, especially 1 to 3 mg/kg. As anti-pneumonia drugs, dosage unit forms such as dragees or capsules for oral administration or ampoules for injections, each containing of from 50 to 200 mg of one or a mixture of active substances, are preferred. Such dosage units are generally administered once to three times, eventually five times, daily depending on the condition of the patient.

For oral administration, there may be used in particular tablets, dragees, capsules, powders or granules, which contain the mixture of the active substances together with the usual excipients and adjuvants such as starch, cellulose powder, talcum, magnesium stearate, sugar, gelatin, calcium carbonate, finely divided silicic acid, carboxymethyl cellulose or similar substances.

For parental administration, in particular for intra-muscular injections, there may be used sterile suspensions, for example oily suspensions prepared with the use of fatty oils such as olive oil, sesame oil, peanut oil, castor oil or a synthetic triglyceride, optionally with simultaneous use of surface-active agents such as sorbitan fatty acid esters. Furthermore, aqueous suspensions may be prepared, for example, with the use of ethoxylated sorbitan fatty acid esters, optionally with addition of thickeners such as polyethylene glycol or carboxymethyl cellulose.

To test the efficiency of the anti-pneumonia compositions according to the invention, aqueous suspensions thereof are administered to rats infected with P. carinii by using an esophagal sound. Rats have the same DHFR amino acid sequence as human DHFR so that such administration correlates readily to administration to humans. For this purpose, the active ingredients are dissolved in water or in another suitable solvent or suspended in 2% aqueous Tylose suspension by adding 100 mg of the composition to be tested to 1 ml of the Tylose suspension. The concentration of active substances to be tested is adjusted by the addition of water. A dose of 0.5 ml per rat per 20 9 body weight provides a useful initial dose, which can be modified according to the effect that it achieves. Prior to administration, the suspensions are treated by ultra-sound to attain a uniform dispersion of the active substances.

Compositions of the invention can be used either for chronic or acute treatment or for prophylaxis. Long-acting forms of triazines, such as the biguanide precursors or pamoate salts described above are particularly preferred for treatment of chronic infection.

### EXAMPLE 1

### Determination of IC50 of para substituted phenyl triazines for P. carinii DHFR at 100 µM DHF

Various 4,6-diamino-1-(p-substituted-pheny1)1,2-dihydro-2,2-dimethyl-s-triazines (referred to generally as para substituted phenyl triazines) were assayed for inhibition of P. carinii DHFR activity. The compounds were dissolved at various concentrations (12.25 mM to 50 mM) in water. A Gilson autosampler (Model 401/232) was programmed to perform 8 serial 5-fold dilutions of each compound into the wells of a microtiter plate (total volume of each dilution was equal to 125 µl). For controls of total P. carinii DHFR activity, 125 µl water was dispensed into the well. The following components were added manually to the resulting 125-ml solutions: 25 µl of 10 mg/ml BSA (in water), 12.5 µl 2 mM NADPH (in water), and 62.55 µl 400 µM DHF in 4 x buffer (200 mM tes, 300 mM BME, 4 mM EDTA, pH 7.0). The microtiter plate was shaken well on a Titertek shaker for 1 minute. Finally 10 µl of 25 nM P. carinii DHFR (in 5O mM tes, 5 mM DTT, 1 mM EDTA, 1 mg/ml BSA) was added, and the microtiter plate was, again, shaken well. The final assay conditions were 100 µM NADPH, 100 µM DHF, 1 nM P. carinii DHFR, and .078 µM to 25 nM para substituted phenyl triazine. The rate of decrease in absorbance at 340 nm was measured in a Thermo-max microtiter plate reader (Molecular Devices) at 25 C.

The activity of each compound was expressed as a percentage of total P. carinii DHFR activity. A graph of % total activity vs. log of concentration of para-substituted phenyl triazine was plotted (see the Figure) and be used to determine an 50IC for each compound and, together with the Km of DHF (0.4 µM) was used to calculate Kᵢ values.

**TABLE 1**

| Para Substituent | IC5O (100 µM DHF) | Kᵢ |
|---|---|---|
| -Cl | 20 µM | 70 nM |
| -CF₃ | 25 µM | 87.5 nM |
| -CH₃ | 30 µM | 105 nM |
| -OCH₃ | 90 µM | 315 nM |
| -OH | 250 µM | 875 nM |
| -CONH₂ | > 8333 µM | > 2900 nM |
| -NO₂ | > 8333 µM | > 2900 nM |

### EXAMPLE 2

### Determination of IC50 of substituted phenyl triazines for P. carinii DHFR and human DHFR

Various 4,6-diamino-1-(substituted-phenyl)1,2-dihydro-2,2-dimethyl-s-triazines (referred to generally as substituted phenyl triazines) were assayed for inhibition of P. carinii and human DHFR activity. The compounds were dissolved at various concentrations (12.25 mM to 50 mM) in water. A Gilson autosampler (Model 401/232) was programmed to perform 8 serial 5-fold dilutions of each compound into the wells of a microtiter plate (total volume of each dilution was equal to 125 µl). For controls of total P. carinii DHFR activity, 125 µl water was dispensed into the well. The following components were added manually to the resulting 125-ml solutions: 25 µl of 10 mg/ml BSA (in water), 12.5 µl 2 mM NADPH (in water), and 62.55 µl, 100 µM DHF in 4 x buffer (200 mM tes, 300 mM BME, 4 mM EDTA, pH 7.0). The microtiter plate was shaken well on a Titertek shaker for 1 minute. Finally 10 µl of 25 nM P. carinii DHFR (in 50 mM tes, 5 mM DTT, 1 mM EDTA, 1 mg/ml BSA) was added, and the microtiter plate was, again, shaken well. The final assay conditions were 100 µM NADPH, 25 µM DHF, 0.42 nM P. carinii DHFR, and .078 µM to 25 nM substituted phenyl triazine. The rate of decrease in absorbance at 340 nm was measured in a Thermo-max microtiter plate reader (Molecular Devices) at 25 C.

In a similar manner, assays were carried out with recombinant human DHFR but with a final human DHFR concentration of 2 nM.

The activity of each compound was expressed as a percentage of total DHFR activity. A graph of % total activity vs. log of concentration of substituted phenyl triazine was plotted and used to determine an 50IC for each compound (at 100 µM or 25 µM). Together with the Km of DHF (0.35 µM), the IC₅₀ (at 25 µM) was used to calculate Kᵢ values.

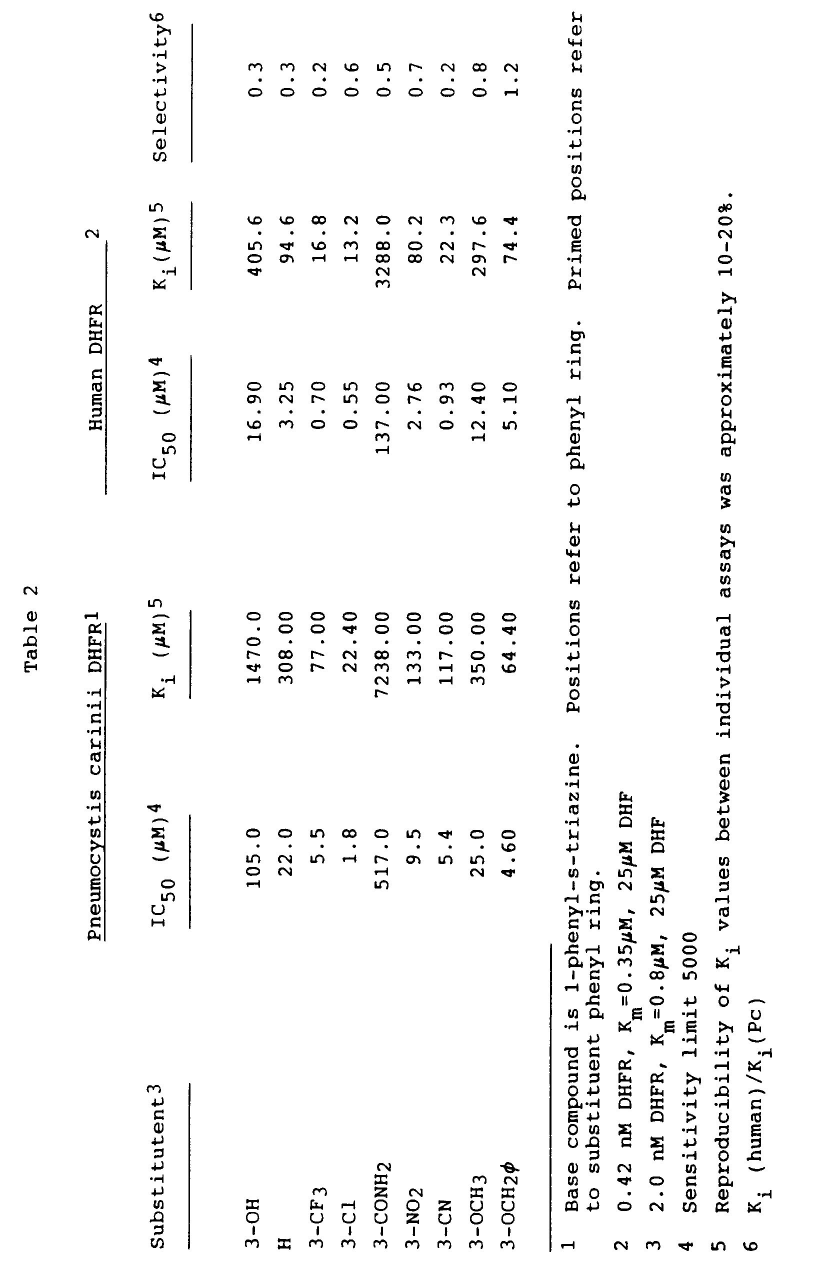

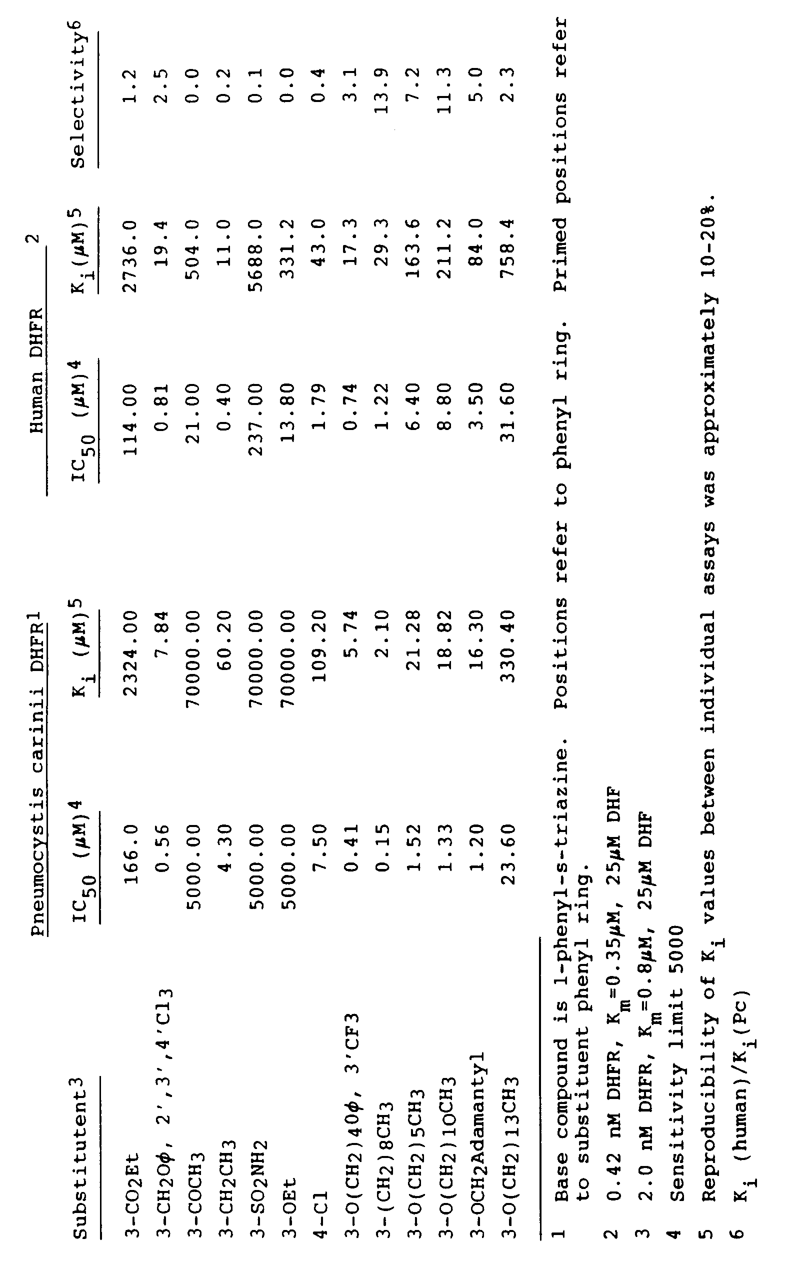

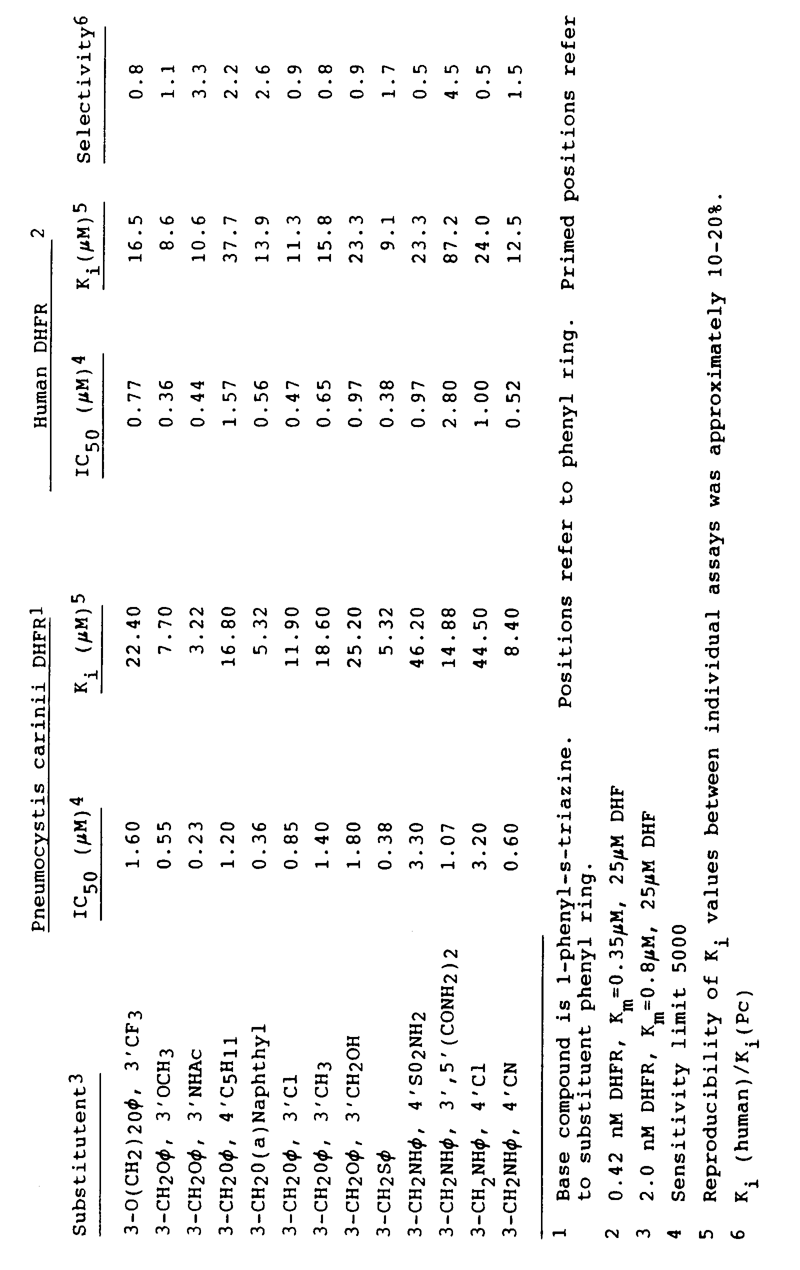

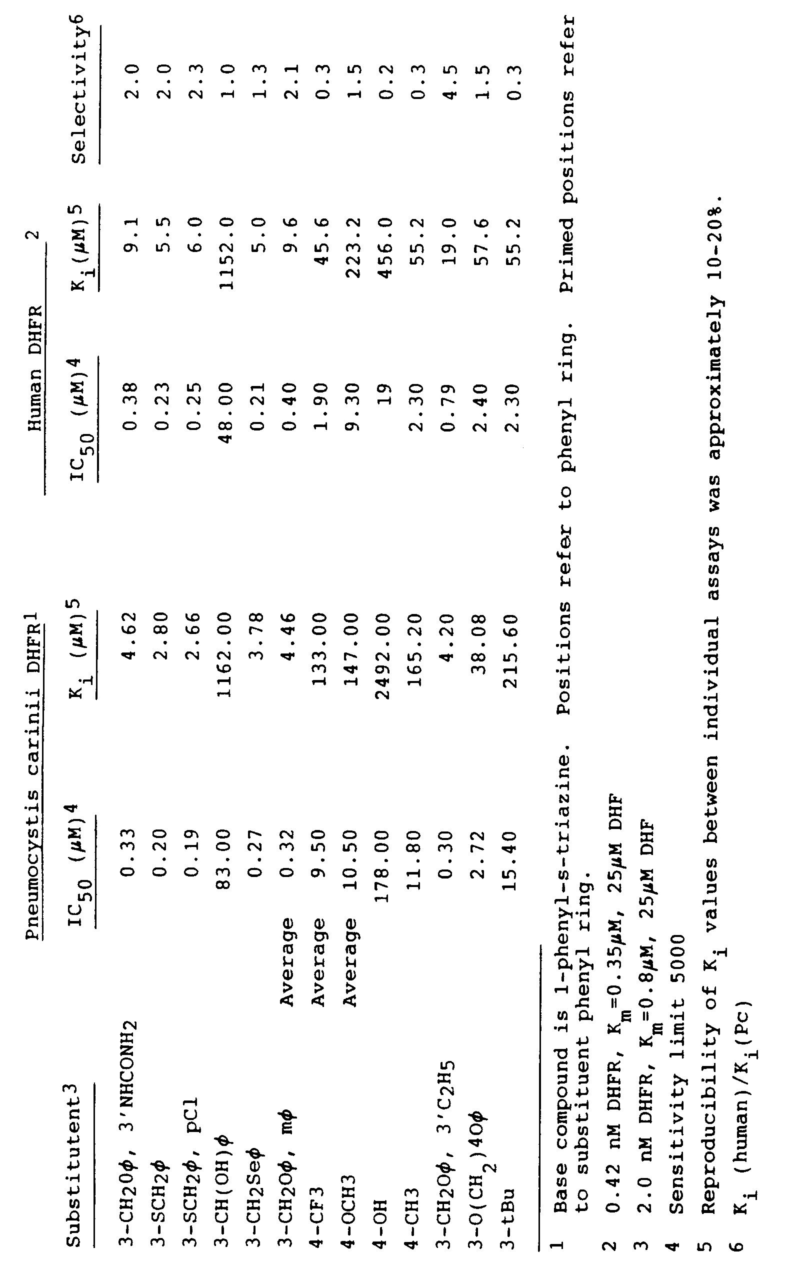

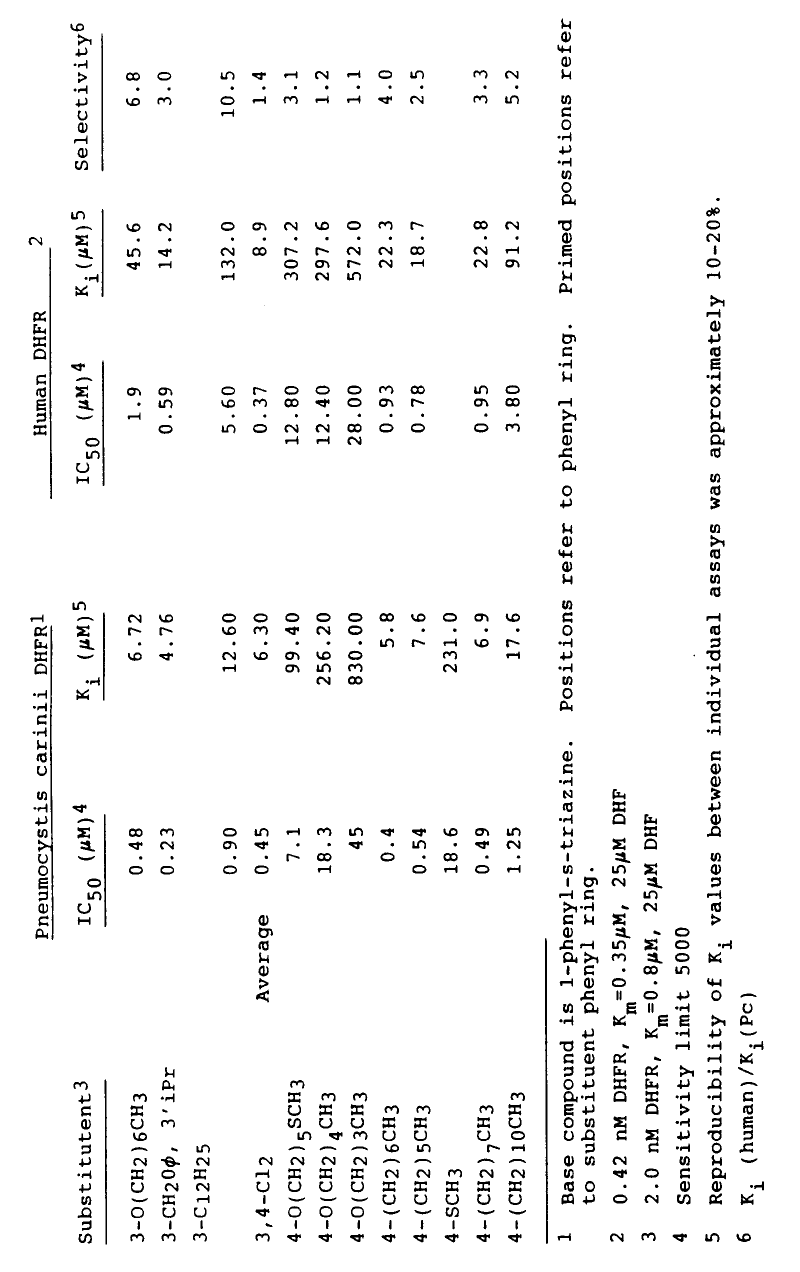

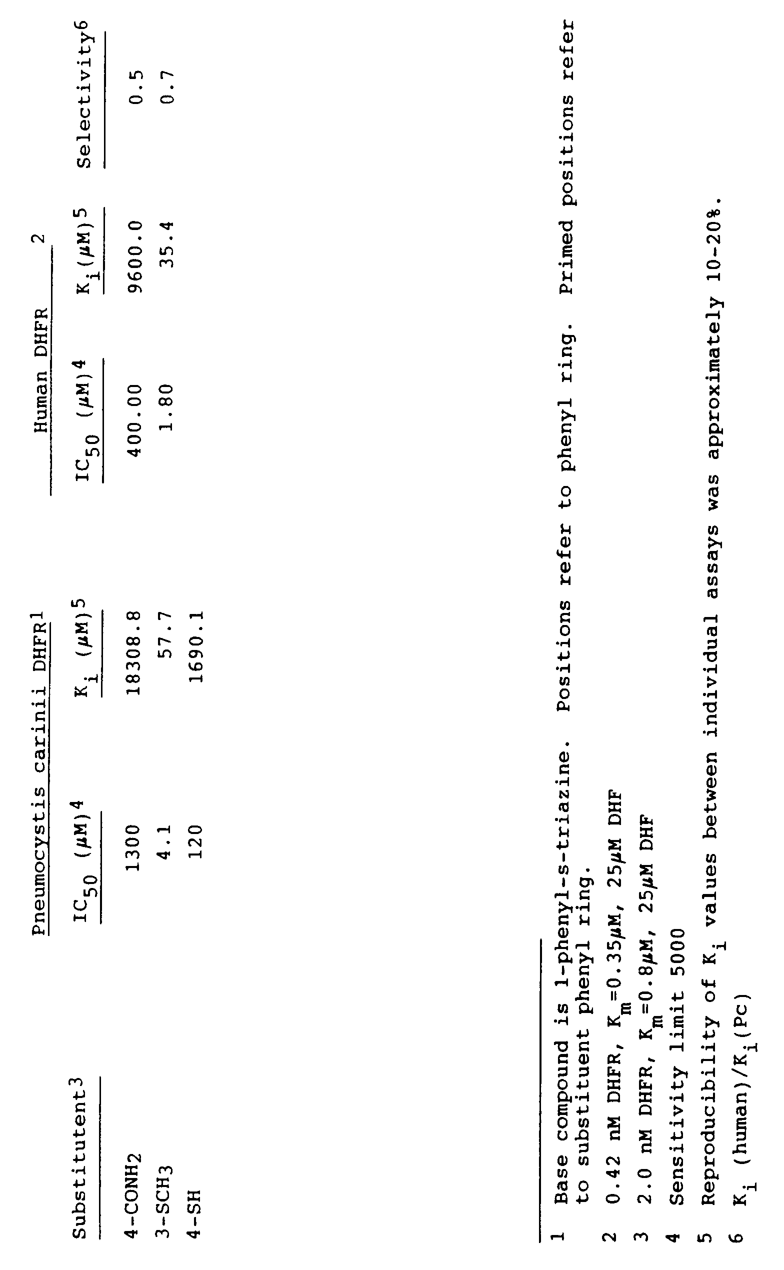

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. Use of a 1-phenyl-s-triazine for the manufacture of a medicament for inhibiting growth of Pneumocystis carinii in a mammalian host.

2. The use of Claim 1, wherein said triazine is a 4,6-diamino-1,2-dihydro-1-phenyl-s-triazine.

3. The use of Claim 1, wherein said triazine has a formula: 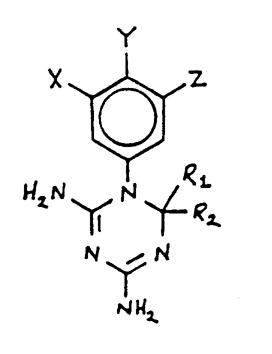 wherein:
X,Y, and Z are independently selected from the group consisting of hydrogen, hydroxy, halogen, lower alkyl, aryl lower alkyl, lower alkylthio, lower alkyloxy, and trifluoromethyl or one of X and Y is selected from the group consisting of CO₂R (where R is lower alkyl), -OCH₂Cl, -OCH₃, and a radical containing at least 3 carbon atoms and being of the formula -A₁-(CH₂)ₙ-A₂-R₃, wherein A₁ is a bond, O, or S; n is an integer from 1 to 12 inclusive; A₂ is a bond, O, S, Se, or NH; and R₃ is selected from the group consisting of methyl, adamantyl, naphthyl, phenyl, and phenyl substituted with 1 to 3 radicals selected from the group consisting of halo, lower alkyl of 1 to 6 carbon atoms, trifluoromethyl, lower alkoxy, cyano, acetamido, ureido, phenyl, and amido; R1 is lower alkyl; and
R2 is hydrogen or methyl;
or said triazine is an amide or acid addition salt formed by reaction of an organic or inorganic acid with a triazine of said formula.

4. The use of Claim 3, wherein said Y is hydrogen, halogen, methyl, methoxy, or trifluoromethyl.

5. The use of Claim 3, wherein at least one of X, Y, and Z is hydrogen.

6. The use of Claim 5, wherein Y is chloro, halogen, methyl, methoxy, or trifluoromethyl.

7. The use of Claim 6, wherein X and Z are hydrogen.

8. The use of Claim 7, wherein R1 and R2 are both methyl.

9. The use of Claim 3, wherein said triazine is a salt of said formula.

10. The use of Claim 9, wherein said salt is a pamoic acid addition salt.

11. Use of a 1-phenyl-s-triazine and at least one other compound able (1) to potentiate activity of a dihydrofolate reductase inhibitor in Pneumocystis carinii or (2) to counter the action of a dihydrofolate reductase inhibitor in said mammalian host for the manufacture of a combined preparation for simultaneous, separate or sequential use in inhibiting growth of Pneumocystis carinii in a mammalian host.

12. The use of Claim 11, wherein said other compound is a sulfonamide.

13. The use of Claim 11, wherein said other compound is a source of reduced folate.

14. The use of Claim 13, wherein said source of reduced folate is citrovorum factor.

15. Use of a precursor of a 1-phenyl-s-triazine for the manufacture of a medicament for inhibiting growth of Pneumocystis carinii in a mammalian host, said precursor being converted to a 1-phenyl-s-triazine in vivo after being administered to said host.

16. The use of Claim 15, wherein the precursor is a biguanide of the formula: 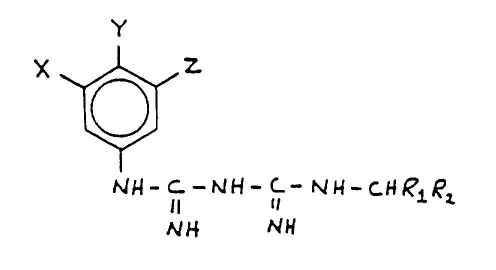 in which X, Y, Z, R1, and R 2 have the meaning defined in Claim 3.

17. The use of Claim 15, wherein said precursor is proguanil.

18. The use according to any of the claims 1 to 17, wherein said host is a human.

19. The use of Claim 18, wherein said 1-phenyl-s-triazine has a selectivity for P. carinii DHFR relative to human DHFR of at least 0.9.

20. The use of Claim 19, wherein said selectivity is at least 2.0.

21. Use according to any of the Claims 1 to 20 for the manufacture of a medicament for oral ingestion.

## Patentansprüche

1. Verwendung eines 1-Phenyl-s-triazins zur Herstellung eines Medikamentes zur Inhibition des Wachstums von Pneumocystis carinii in einem Säugetierwirt.

2. Verwendung nach Anspruch 1, wobei das Triazin ein 4,6-Diamino-1,2-dihydro-1-phenyl-s-triazin ist.

3. Verwendung nach Anspruch 1, wobei das Triazin eine Formel hat: 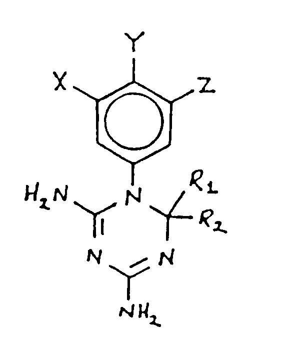 wobei:
X, Y und Z unabhängig aus der Gruppe, bestehend aus Wasserstoffatom, Hydroxylgruppe, Halogenatom, niederer Alkyl-, arylierter niederer Alkyl-, niederer Alkylthio-, niederer Alkyloxyrest und Trifluormethylgruppe ausgewählt werden; oder einer der Reste X und Y aus der Gruppe ausgewählt wird bestehend aus CO₂R (wobei R ein niederer Alkylrest ist), -OCH₂Cl, -OCH₃ und einem Rest der Formel -A₁-(CH₂)ₙ-A₂-R₃ mit wenigstens 3 Kohlenstoffatomen, wobei A₁ eine Bindung, O oder S ist; n ist eine Integerzahl von 1 bis 12 einschließlich; A₂ ist eine Bindung, O, S, Se oder NH; und R₃ wird aus der Gruppe ausgewählt, die aus der Methyl-, Adamantyl-, Naphthyl-, Phenylgruppe und der mit 1 bis 3 Resten, die aus der Gruppe bestehend aus Halogenkohlenwasserstoff, niederen Alkylresten mit 1 bis 6 Kohlenstoffatomen, Trifluormethyl-, niederen Alkoxy-, Cyano-, Acetamido-, Ureido-, Phenyl- und Amidogruppe ausgewählt werden, substituierten Phenylgruppe besteht;
R₁ ein niederer Alkylrest ist; und
R₂ ein Wasserstoffatom oder eine Methylgruppe ist;
oder das Triazin ist ein Amid oder ein Säureadditionssalz, das durch Reaktion einer organischen oder anorganischen Säure mit einem Triazin dieser Formel gebildet wurde.

4. Verwendung nach Anspruch 3, wobei der Rest Y ein Wasserstoffatom, ein Halogenatom, eine Methyl-, Methoxy- oder Trifluormethylgruppe ist.

5. Verwendung nach Anspruch 3, wobei wenigstens einer der Reste X, Y und Z ein Wasserstoffatom ist.

6. Verwendung nach Anspruch 5, wobei der Rest Y ein Chlor- oder ein Halogenatom, oder eine Methyl-, Methoxy- oder Trifluormethylgruppe ist.

7. Verwendung nach Anspruch 6, wobei die Reste X und Z Wasserstoffatome sind.

8. Verwendung nach Anspruch 7, wobei die Reste R₁ und R₂ beide Methylgruppen sind.

9. Verwendung nach Anspruch 3, wobei das Triazin ein Salz besagter Formel ist.

10. Verwendung nach Anspruch 9, wobei das Salz ein Additionssalz der Pamoasäure ist.

11. Verwendung eines 1-Phenyl-s-triazins und wenigstens einer anderen Verbindung, die in der Lage ist, (1) die Aktivität eines Dihydrofolat-Reduktaseinhibitors in Pneumocystis carinii zu verstärken oder (2) der Wirkung eines Dihydrofolat-Reduktaseinhibitors in dem Säugetierwirt entgegenzuwirken, zur Herstellung einer kombinierten Zubereitung zur gleichzeitigen separaten oder sequentiellen Verwendung beim Inhibieren des Wachstums von Pneumocystis carinii in einem Säugetierwirt.

12. Verwendung nach Anspruch 11, wobei die andere Verbindung ein Sulfonamid ist.

13. Verwendung nach Anspruch 11, wobei die andere Verbindung eine Quelle für reduziertes Folat ist.

14. Verwendung nach Anspruch 13, wobei die Quelle für das reduzierte Folat Citrovorumfaktor ist.

15. Verwendung eines Präkursors eines 1-Phenyl-s-triazins zur Herstellung eines Medikaments zur Inhibierung des Wachstums von Pneumocystis carinii in einem Säugetierwirt, wobei dieser Präkursor sich in vivo zu einem 1-Phenyl-s-triazin umwandelt, nachdem er dem Wirt verabreicht wurde.

16. Verwendung nach Anspruch 15, wobei der Präkursor ein Biguanid der Formel ist: 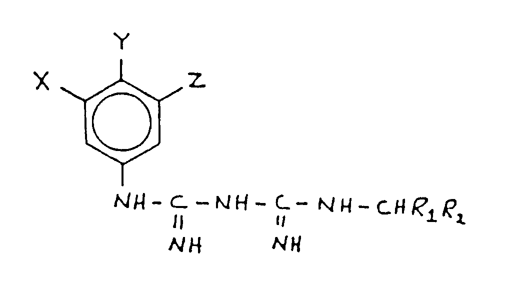 in der die Reste X, Y, Z, R₁ und R₂ die in Anspruch 3 definierte Bedeutung haben.

17. Verwendung nach Anspruch 15, wobei der Präkursor ein Proguanil ist.

18. Die Verwendung gemäß einem der Ansprüche 1 bis 17, wobei der Wirt ein Mensch ist.

19. Verwendung nach Anspruch 18, wobei das 1-Phenyl-s-triazin für P. carinii DHFR eine Selektivität relativ zum menschlichen DHFR von wenigstens 0,9 aufweist.

20. Verwendung nach Anspruch 19, wobei diese Selektivität wenigstens 2,0 beträgt.

21. Verwendung nach einem der Ansprüche 1 bis 20 für die Herstellung eines Medikaments zur oralen Einnahme.

## Revendications

1. Utilisation d'une 1-phényl-s-triazine pour la fabrication d'un médicament pour inhiber la croissance de *Pneumocystis carinii* chez un hôte mammifère.

2. Utilisation selon la revendication 1, dans laquelle cette triazine est une 4,6-diamino-1,2-dihydro-1-phényl-s-triazine.

3. Utilisation selon la revendication 1, dans laquelle cette triazine a la formule : 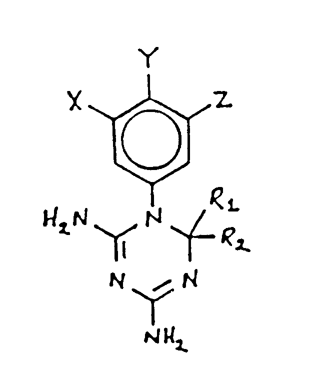 dans laquelle :
X, Y et Z sont indépendamment choisis parmi le groupe comportant un atome d'hydrogène, un groupe hydroxy, un atome d'halogène, un groupe alkyle inférieur, un groupe arylalkyle inférieur, un groupe alkylthio inférieur, un groupe alkyloxy inférieur et un groupe trifluorométhyle ou bien l'un des X et Y est choisi parmi le groupe comportant le radical CO₂R, dans lequel R est un groupe alkyle inférieur, le radical ðOCH₂Cl, le radical ðOCH₃ et un radical contenant au moins 3 atomes de carbone et étant représenté par la formule ðA₁ð(CH₂)ₙðA₂ðR₃, dans laquelle A₁ est une liaison, un atome d'oxygène ou de soufre; n est un entier de 1 à 12 inclus; A₂ est une liaison, un atome d'oxygène, de soufre, de sélénium ou le radical NH; et
R₃ est choisi parmi le groupe comportant un groupe méthyle, un groupe adamantyle, un groupe naphtyle, un groupe phényle et un groupe phényle substitué avec 1 à 3 radicaux choisis parmi le groupe comportant un atome d'halogène, un groupe alkyle inférieur de 1 a 6 atomes de carbone, un groupe trifluorométhyle, un groupe alcoxy inférieur, un groupe cyano, un groupe acétamido, un groupe uréido, un groupe phényle et un groupe amido;
R₁ est un groupe alkyle inférieur; et
R₂ est un atome d'hydrogène ou un groupe méthyle;
ou bien cette triazine est un amide ou un sel d'addition d'acide formé par réaction d'un acide organique ou inorganique avec une triazine de cette formule.

4. Utilisation selon la revendication 3, dans laquelle Y est un atome d'hydrogène, d'halogène,un groupe méthyle, un groupe méthoxy ou un groupe trifluorométhyle.

5. Utilisation selon la revendication 3, dans laquelle l'un au moins des X, Y et Z est un atome d'hydrogène.

6. Utilisation selon la revendication 5, dans laquelle Y est un atome de chlore, un atome d'halogène, un groupe méthyle, un groupe méthoxy ou un groupe trifluorométhyle.

7. Utilisation selon la revendication 6, dans laquelle X et Z sont chacun un atome d'hydrogène.

8. Utilisation selon la revendication 7, dans laquelle R₁ et R₂ sont tous deux un groupe méthyle.

9. Utilisation selon la revendication 3, dans laquelle cette triazine est un sel de cette formule.

10. Utilisation selon la revendication 9, dans laquelle ce sel est un sel d'addition d'acide pamoïque.

11. Utilisation d'une 1-phényl-s-triazine et d'au moins un autre composé capable (1) de potentialiser l'activité d'un inhibiteur de dihydrofolate réductase chez *Pneumocystis carinii* ou (2) de contrer l'action d'un inhibiteur de dihydrofolate réductase chez cet hôte mammifère pour la fabrication d'une préparation combinée pour l'utilisation simultanée, séparée ou successive dans le but d'inhiber la croissance de *Pneumocystis carinii* chez un hôte mammifère.

12. Utilisation selon la revendication 11, dans laquelle cet autre composé est un sulfonamide.

13. Utilisation selon la revendication 11, dans laquelle cet autre composé est une source de folate réduit.

14. Utilisation selon la revendication 11, dans laquelle cette source de folate réduit est un facteur citrovorum.

15. Utilisation d'un précurseur d'une 1-phényl-s-triazine pour la fabrication d'un médicament pour inhiber la croissance de *Pneumocystis carinii* chez un hôte mammifère, ce précurseur étant converti en une 1-phényl-s-triazine *in vivo* après avoir été administré à cet hôte.

16. Utilisation selon la revendication 15, dans laquelle le précurseur est un biguanide de formule : 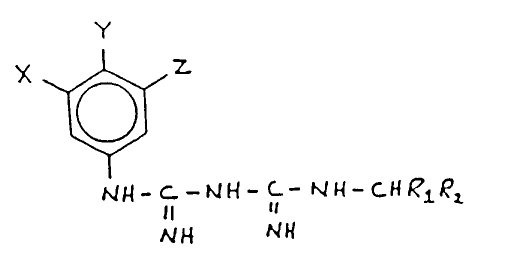 dans laquelle X, Y, Z, R₁ et R₂ sont tels que définis dans la revendication 3.

17. Utilisation selon la revendication 15, dans laquelle ce précurseur est le proguanile.

18. Utilisation selon l'une quelconque des revendications 1 à 17, dans laquelle cet hôte est un être humain.

19. Utilisation selon la revendication 18, dans laquelle cette 1-phényl-s-triazine présente une sélectivité d'au moins 0,9 pour la DHFR de *P. carinii* par rapport à la DHFR humaine.

20. Utilisation selon la revendication 19, dans laquelle ladite sélectivité est d'au moins 2,0.

21. Utilisation selon l'une quelconque des revendications 1 à 20, pour la fabrication d'un médicament destiné à l'administration orale.
